(19) Europäisches Patentamt

European Patent Office

Office européen des brevets

(11) **EP 1 726 258 A2**

(12) **EUROPEAN PATENT APPLICATION**

(43) Date of publication:
**29.11.2006 Bulletin 2006/48**

(51) Int Cl.:
*A61B 5/097* (2006.01)    *G01N 33/00* (2006.01)

(21) Application number: **05018413.4**

(22) Date of filing: **24.08.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **25.05.2005 JP 2005152810**

(71) Applicant: **HITACHI, LTD.**
**Chiyoda-ku**
**Tokyo 100-8280 (JP)**

(72) Inventors:
• **Yamada, Masuyoshi**
**Hitachi Ltd - Intel. Prop. Off.**
**Chiyoda-ku**
**Tokyo 100-8220 (JP)**
• **Suga, Masao,**
**Hitachi Ltd - Intell. Prop. Off.**
**Chiyoda-ku**
**Tokyo 100-8220 (JP)**
• **Waki, Izumi,**
**Hitachi Ltd - Intell. Prop. Off.**
**Chiyoda-ku**
**Tokyo 100-8220 (JP)**

(74) Representative: **Strehl Schübel-Hopf & Partner**
**Maximilianstrasse 54**
**80538 München (DE)**

(54) **Exhaled-air filter, collecting apparatus, analyzing system and method**

(57) For collecting a component of an exhaled air, a filter (1) comprises an adsorbent (211) for adsorbing the component of the exhaled air, and a collector body (212) including a passage hole (213) in which the adsorbent is contained and through which the exhaled air is capable of passing.

FIG. 2A

**Description**

BACKGROUND OF THE INVENTION

The present invention relates to an exhaled air filter, exhaled air collecting apparatus, exhaled air analyzing system and exhaled air analyzing method, for analyzing a component in an exhaled air of mammals.

JP-A-10-206293 (claim 1 and paragraph 0020) discloses that an adsorbent and heating element are contained in a glass tube, an exhaled air passes through the tube to adsorb a component of the exhaled gas in the adsorbent, the heating element is irradiated with microwave to be heated so that the component vaporizes from the adsorbent, and the vaporized component is analyzed.

JP-A-2003-171317 (claim 5 and paragraphs 0047-0053) discloses that the exhaled air is received in a bag, and the bag is connected to a mass spectrometer to analyze the component of the exhaled air.

JP-A-8-51098 (claim 2) discloses that an exhaled air collecting apparatus has a valve to collect only alveolar exhaled air.

BRIEF SUMMARY OF THE INVENTION

An object of the present invention is to provide an exhaled air filter, exhaled air collecting apparatus, exhaled analyzing system and exhaled air analyzing method, for analyzing a component in an exhaled air of mammals, by which a physical burden of a test subject on collecting the component in the exhaled air can be decreased, and an efficiency of collecting or adsorbing the component in the exhaled air can be improved.

According to the invention, a filter for collecting a component of an exhaled air, comprises, an adsorbent for adsorbing the component of the exhaled air, and a collector body including a passage hole in which the adsorbent is contained and through which the exhaled air is capable of passing.

It is preferable for decreasing the physical burden of the test subject on collecting the component in the exhaled air that when a total amount of volume of the adsorbent is V, a total amount of cross sectional area of the passage hole is A, and a length of the passage hole is L, $(AL - V)^2/L^3 \geq 0.003mm^3$, and $V / AL \geq 0.3$.

If the collector body has a pair of meshes covering respective opening ends (opposite to each other in a flow direction of the exhaled air) of the passage hole to prevent the adsorbent from moving out of the passage hole and to allow the exhaled air to pass through the meshes, a flow resistance for the exhaled gas can be kept constant with a correct thickness of the meshes.

If the collector body includes a plurality of the passage holes arranged in parallel in a flow direction of the exhaled air (and juxtaposed to each other in a radial direction perpendicular to the flow direction) so that the passage holes are capable of passing respective parts of the exhaled air in parallel, the flow resistance for the exhaled gas can be decreased to decrease the physical burden of the test subject on collecting the component in the exhaled air. If the collector body has a partition wall between the passage holes to prevent the parts of the exhaled air in the respective passage holes from fluidly communicating with each other in a direction perpendicular to the flow direction of the exhaled air in the collector body and to restrain the adsorbent in each of the passage holes from being gathered or condensed to one radial side of the passage hole in by weight of the adsorbent in the direction perpendicular to the flow direction of the exhaled air in the collector body or a longitudinal direction of the passage hole so that a clearance between an inner peripheral surface of the passage hole and the adsorbent is restrained from forming in the passage hole and the exhaled gas is restrained from passing through the clearance in the longitudinal direction of the passage hole without passing through the adsorbent while the exhaled gas can sufficiently contact the adsorbent so that the component in the exhaled gas can be efficiently adsorbed on the adsorbent, the flow resistance for the exhaled air can be kept constant and low to decrease the physical burden of the test subject on collecting the component in the exhaled air, and the efficiency of collecting or adsorbing the component in the exhaled air can be improved by preventing the absorbent (granular) from being condensed by the weight of the absorbent itself. If the collector body has a pair of meshes covering respective opening ends of each of the passage holes to prevent the adsorbent from moving out of the passage hole and to allow the exhaled air to pass through the meshes, and each of the meshes extends monolithically to cover the opening ends of the passage holes (while being prevented from extending into the passage holes), a fluidal communication over the opening ends of the passage holes through each of the meshes is obtained so that the exhaled air can be distributed correctly evenly to the opening ends of the passage holes, the passage holes can be treated as a monolithic one piece unit, and a temperature of the adsorbents in the passage holes and a temperature of the exhaled airs taken into the passage holes respectively can be kept evenly over the passage holes, that is, a difference in inner (adsorbent) temperature between the passage holes is minimized and a difference in exhaled air taken into the passage hole between the passage holes is minimized.

If the collector body (including the meshes and inner peripheral surfaces of the passage holes) is metallic, a temperature of the adsorbent in the passage hole can be kept evenly in each of longitudinal and radial directions of the passage hole, and/or a temperature of the adsorbents in the passage holes can be kept evenly over the passage holes, that is a difference in inner (adsorbent) temperature between the passage holes is minimized, (while little amount of impurities are emitted from the material of the collector body and back-ground noise level is reduced).

It is preferable that the adsorbent includes a carbon type adsorbent.

According to the invention, an apparatus for collecting an exhaled air, comprises, an inlet for receiving the exhaled air from a test subject, and the filter as recited in claims 1-11 for collecting a component of the exhaled air, wherein the filter includes an adsorbent for adsorbing the component of the exhaled air, and a collector body including a passage hole in which the adsorbent is contained and through which the exhaled air is capable of passing.

The apparatus may further comprise a flow meter (preferably mass flow meter) for measuring a flow rate of the exhaled air passing through the passage hole to measure a total amount of the exhaled air passing through the passage hole to be compared with a total amount of the removed and measured component of the exhaled air so that a concentration of the component in the exhaled gas is calculated. The apparatus may further comprise a suction pump fluidly connected to one of opening ends of the passage hole to accelerate a flow of the exhaled air from the other one of the opening ends toward the one of the opening ends so that the physical burden of the test subject is decreased. The apparatus may further comprise a heater for heating the adsorbent to remove or vaporize or reproduce the component from the adsorbent, and an analyzer for analyzing the component removed or vaporizes or reproduced from the adsorbent. It is preferable that the heater is capable of generating a radiant heat for irradiating the filter. The analyzer may include at least one of a mass spectrometer and a gas chromatograph.

According to the invention, a method for analyzing an exhaled air, in an apparatus for collecting an exhaled air, including an inlet for receiving the exhaled air from a test subject, and a filter for collecting a component of the exhaled air, wherein the filter includes an adsorbent for adsorbing the component of the exhaled air, and a collector body including a passage hole in which the adsorbent is contained and through which the exhaled air is capable of passing, comprising the steps of:

receiving the exhaled air at the inlet so that the exhaled air is allowed to pass through the passage hole, heating the adsorbent to remove or vaporize or reproduce the component from the adsorbent, and analyzing the removed or vaporized or reproduced component.

It is preferable for collecting and measuring the component of the exhaled air of low volatility and preventing the component of the exhaled air of high volatility from being adsorbed by the adsorbent that the method further comprises the step of heating the adsorbent to a temperature (more than an estimated temperature of the exhaled air to be received at the inlet) preventing the component to be measured from being removed or vaporized or reproduced from the adsorbent during the adsorption and making another component of high volatility not to be measured or detected be restrained from being adsorbed by the adsorbent before the step of receiving the exhaled air at the inlet, because the component can be analyzed in high sensitivity by heating the adsorbent to the temperature preventing the component to be measured from being removed or vaporized or reproduced from the adsorbent during the adsorption to make another component of high volatility not to be measured or detected be restrained from being adsorbed by the adsorbent.

It is preferable for collecting and measuring the component of the exhaled air of low volatility and preventing the component of the exhaled air of high volatility from being adsorbed by the adsorbent that a temperature of the adsorbent is kept at a temperature more than a temperature of the exhaled air received at the inlet, in the step of receiving the exhaled air, the temperature preventing the component to be measured from being removed or vaporized or reproduced from the adsorbent during the adsorption and making another component of high volatility not to be measured or detected be restrained from being adsorbed by the adsorbent, because the component can be analyzed in high sensitivity by heating the adsorbent to heat the exhaled air to the temperature preventing the component of low volatility to be measured from being removed or vaporized or reproduced from the adsorbent during the adsorption to make another component of high volatility not to be measured or detected be restrained from being adsorbed by the adsorbent.

It is preferable for collecting and measuring the component of the exhaled air of high volatility, that a temperature of the adsorbent is kept less than a temperature of the exhaled air received at the inlet, in the step of receiving the exhaled air, because the component of high volatility can be condensed or liquefied by cooling the adsorbent to cool the exhaled air in the adsorbent during the adsorption to make the component effectively be adsorbed by a surface of the adsorbent. The temperature of the adsorbent may be kept less than the temperature of the exhaled air received at the inlet in the step of receiving the exhaled air, after the step of heating the adsorbent, so that the exhaled air of high volatility is prevented or restrained from being adsorbed by the adsorbent before the step of receiving the exhaled air.

Other objects, features and advantages of the invention will become apparent from the following description of the embodiments of the invention taken in conjunction with the accompanying drawings.

BRIEF DESCRIPTION OF THE SEVERAL VIEWS OF THE DRAWINGS

Fig. 1 is a schematic view showing an exhaled air collecting apparatus as an embodiment of the invention.
Fig. 2a is a schematic cross sectional view showing a filter of the invention.
Fig. 2b is a schematic cross sectional view taken along IIb-IIb in fig. 2a.
Fig. 3 is a schematic oblique projection view showing an exhaled air analyzing system.
Fig. 4 is a flow chart of handling process for a component of the exhaled gas.

DETAILED DESCRIPTION OF THE INVENTION

An exhaled cir collecting filter, exhaled air collecting apparatus and exhaled air analyzing system as embodiments of the invention will be described with making reference to the drawings.

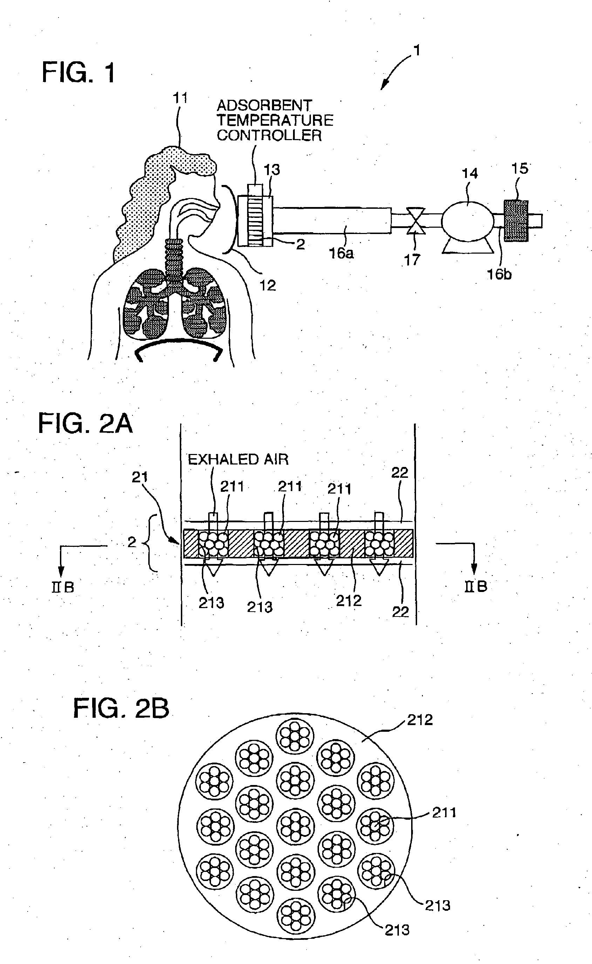

Fig. 1 is a schematic view showing the exhaled air collecting apparatus as the embodiment.
As shown in fig. 1, the exhaled air collecting apparatus 1 has an exhaled air receiving part 12 for receiving an exhaled air of a test subject 11, and an exhaled air collecting part 13 connected to the exhaled air receiving part 12 and including an adsorbing part (an exhaled air collecting filter) 2 for collecting a component of the exhaled air. Further, the exhaled air collecting apparatus 1 has a suction pump 14 arranged at a downstream side of the exhaled air collecting part 13 and connected to the exhaled air collecting part 13 through a pipe 16a so that a pressure at the downstream side of the exhaled air collecting part 13 and the pipe 16a is decreased, and a flow meter 15 arranged at a downstream side of the suction pump 14 and connected to the suction pump 14 through a pipe 16b so that an amount of the collected exhaled air is measured. Hexe, the amount of the exhaled air is an amount of the exhaled air flowing in a unit time period. Incidentally, the flow meter 15 may be arranged on the pipe 16a at an upstream side of the suction pump 14. Further, the exhaled air collecting apparatus 1 may have a valve 17 arranged on the pipe 16a to adjust the amount of the exhaled gas flowing in the pipe 16a.
Incidentally, the exhaled air receiving part 12 has preferably a mask shape covering a mouth, but may have a pipe shape to be fressed in the mouth to receive the exhaled air.
The test subject 11 holds the exhaled air receiving part 12 at the mouth and discharges the exhaled air. The discharged exhaled air is fed to the exhaled air collecting part 13. The component of the exhaled air fed to the exhaled air collecting part 13 is adsorbed in an adsorbent 211 (refer to fig. 2) in the adsorbing part 2 arranged in the exhaled air collecting part 13. Another component not adsorbed by the adsorbent 211 is discharged out of the system through the pipe 16a, valve 17, suction pump 14 and pipe 16b. It is preferable that the suction pump 14 is arranged at the downstream side of the exhaled air collecting part 13 to decrease the pressure at the downstream side of the exhaled air collecting part 13 and the pipe 16a to decrease a load for the test subject 11. Further, the valve 17 may be arranged on the pipe 16a to adjust the amount of the exhaled air flowing in the pipe 16a. Further, it is preferable that the flow meter 15 is arranged at the upstream or downstream side of the suction pump 14 (only the suction pump 14 arranged at the downstream side is shown in the drawings as the embodiment) to detect the amount of the exhaled air discharged to the exhaled air collecting apparatus 1. Since an amount of the component of the exhaled air collected by the adsorbent 211 depends on the amount of the exhaled air, it is important for keeping sampling condition constant that the amount of the exhaled air is measured by the flow meter 15.
Fig. 2 is a view showing a structure of the adsorbing part of the embodiment, fig. 2a is a cross sectional side view of the adsorbent part, and fig. 2b is a cross sectional view taken along A-A in fig. 2a.
Hereafter, the structure of the adsorbing part 2 will be explained with making reference to fig. 2.
As shown in fig. 2a, the adsorbing part 2 has meshes 22 contacting respective surfaces of the adsorbing filter 21. The meshes 22 may be or may not be adhered to the adsorbing filter 21. The adsorbing filter 21 has the adsorbent 211 for adsorbing the component of the exhaled air, and a metallic sheet (collecting part) 212 for holding the adsorbent 211. The metallic sheet 212 has holes 213 extending from a predetermined (upstream) surface to another (downstream) surface. As shown in fig. 2b, the granular adsorbent 211 is contained in the holes 213. A clearance size of the meshes 22 is smaller than a granular diameter of the adsorbent 211, so that the adsorbent 211 is prevented from flowing out of the holes 213. Further, if a periphery of the adsorbing part 2 or collecting filter 21 is hermetically sealed (not shown) to prevent the exhaled air from being discharged from the periphery of the adsorbing part 2, a rate of the exhaled air passing through the holes 213 containing the adsorbent 211 is increased to improve an adsorbing efficiency of the exhaled air. A material of the metallic sheet 212 and meshes 22 is preferably for preventing a gas from being emitted when being heated, for example, stainless steel or the like. The adsorbent 211 may be selected in accordance with the component to be detected, for example, may be carbon type adsorbent. Concretely, for example, TENAX TA® or TENAX GR® of Buchem BV is generally used.
Next, the structure and action of the collecting filter 21 will be explained in detail with making reference to figs.

1 and 2..

The exhaled air receiving part 12 as the mask is attached to the test subject 11 so that the exhaled air of the test subject 11 is discharged to the adsorbing part 2 in the exhaled air collecting part 13. The component of the exhaled air such as hydrocarbon-type matter is selectively adsorbed by the adsorbent 211, and an inert gas such as nitrogen is not adsorbed and is discharged to the downstream side of the adsorbing part 2. When the downstream side of the adsorbing part 2 is of atmospheric temperature, the pressure of the upstream side of the adsorbing part 2 is higher than that of the downstream side thereof due to a pressure loss caused by passage of the exhaled air through the adsorbing part 2, to form a resistance against the discharge of the exhaled from the test subject 11. Therefore, the lower the pressure loss is, the lower the pressure at the upstream side of the adsorbing part 2 is, so that the test subject 11 can discharge without effort the exhaled air. A relationship between the amount of the exhaled air flowing through the adsorbing part 2 and the pressure loss caused thereby is as mentioned below.

When a total amount of cross sectional areas of the holes 213 (hereafter, called as cross sectional area) is A, a length of the holes 213 is L, and a total amount of volumes of the adsorbent 211 in the holes 213 is V, a volume through which the exhaled air is capable of passing in the collecting filter 21 is AL - V. Therefore, an apparent cross sectional area through which the exhaled air is capable of passing is,

$$(AL - V) / L \qquad \text{(formula 1).}$$

Further, the pressure loss P between the upstream and downstream sides of the adsorbing part 2 is

$$P = Q / C \qquad \text{(formula 2),}$$

where, Q: the amount of the exhaled air, C: conductance.

The conductance C of cylindrical tube (under the atmosphere of 20°C) is estimated from

$$C = \{1349 \cdot d^4 / L\} \cdot \{(P_1 + P_2) / 2\} \ [m^3 /s] \qquad \text{(formula 3),}$$

where, d: apparent inner diameter of the cylindrical tube = $2 \cdot ((AL - V) / \pi L)^{1/2}$, $P_1$ : pressure at upstream side of adsorbing part 2, $P_2$ : pressure at downstream side of adsorbing part 2.

In this way, from the total amount volume of the used adsorbent 211, the amount of exhaled air Q and so forth, the pressure loss P can be calculated. Here, when the maximum value of the amount of the exhaled gas during normal breathing is about 4 [1 / min], and the pressure loss between the upstream and downstream sides of the adsorbing part 2 on this time should be not more than 10 [kPa], that is,

$$P = Q / C \leq 10 \times 10^3 \qquad \text{(formula 4),}$$

from an experimental result by the inventors, the load during sampling the exhaled air cannot be felt or is sufficiently or extremely low.

That is, when $P_1 = 110 \times 10^3$ [Pa], $P_2 = 100 \times 10^3$ [Pa], Q = $4 \times 10^{-3} \times 10^5$ /60 [Pa · m3 / s] are incorporated into the formulas 3 and 4, and the formula 3 is incorporated into the formula 4,

$$(AL - V)^2 / L^3 \geq 2.9 \times 10^{-12} \ [m^3] = 2.9 \times 10^{-3} \ [mm^3],$$

so that $(AL - V)^2 / L^3 \geq 0.003$ [mm$^3$] is satisfied to generate the pressure loss not more than 10 [kPa] for preventing the test subject 11 from feeling the load when the test subject 11 discharge the amount of the exhaled air of 4 [1 / min].

On the other hand, for analyzing the component of the exhaled air, it is preferable for the exhaled gas to contact as largely as possible to accelerate the adsorption of the component of the exhaled air. That is, if the above mentioned ratio of between AL and V (a rate in volume of the adsorbent 211 with respect to the holes 213) V / AL is excessively small (few), a major part of the component of the exhaled air does not contact the adsorbent 211 and is discharged to the exterior, so that a sensitive measurement is not obtainable, From the experimental result by the inventors, when V / AL is not less than 80%, an adsorbing efficiency is not less than 90%, but, the adsorbing efficiency decreases to about 50% when V / AL is 30%, and the adsorbing efficiency decreases to about 10% when V / AL is 10%, so that it is preferable that V / AL $\geq$ 30%.

The smaller the particle diameter of the adsorbent 211 is, the greater a surface area thereof per the same volume is, to increase a performance of adsorbing the component of the exhaled air, but the higher the rate in volume of the adsorbent 211 with respect to the holes 213 to increase the pressure loss. Therefore, the holes 213 may have complicated surface shape to increase a surface area of the holes 213, so that if the particle diameter is not changed, the adsorbing efficiency is increased without significant increase of the pressure loss.

If the used amount of the adsorbent 211 is not.changed, it is effective for decreasing the pressure loss between the upstream and downstream sides of the adsorbing part 2 and decreasing a flowing velocity of the air through the collecting filter 21 that a cross sectional area A is increased. But, if the metallic sheet 212 as shown in fig. 2 is not used and the adsorbent 211 is held only between the meshes 22 (that is, a diameter of the hole 213 is increased significantly), the adsorbent 211 moves to one side in each of the holes when the collecting part'2 is moved so that the major part of the exhaled air to be sampled discharged out of the system without contacting the adsorbent 211. As described above, the metallic sheet 212 has the holes 213 is effective for preventing the adsorbent 211 from moving.

As an example of the collecting part 2 as shown in fig. 2, when an inner diameter of each of the holes 213 is $3\times10^{-3}$ [m], a length of the holes 213 is $0.8\times10^{-3}$ [m], a number of the holes is 31, mesh size is about 250$\mu$m, the absorbent 211 is TENAX TA® of Buchem BV® (diameter of about 250$\mu$m), and a total mount of volume of the adsorbent is about $106\times10^{-6}$ [m³], since a total amount of cross sectional areas of the holes 213 without the adsorbent 211 therein is about $219\times10^{-6}$ [m²], a total amount of volumes of the holes 213 is about $219\times10^{-6}\times0.8\times10^{-3} = 175\times10^{-9}$[m3]. Therefore, the rate in volume of the adsorbent with respect to the holes 213 (V / AL) is about 60%. Further, $(AL / V)^2 / L^3$ = $(175\times10^{-9}\ 106\times10^{-9})^2 / (0.8\times10^{-3})^3 \fallingdotseq 9300\times10^{-9}$[m³] = 9300 [mm³], so that both $(AL - V)^2 / L^3 \geq 0.003$ [mm³] and V /Al $\geq$ 0.3(30%) are satisfied.

When actually the collecting filter 21 of the embodiment was used and the exhaled air flowed at 4 [1 / min], the pressure loss was about 1 [kPa] as experimentally confirmed, and sufficiently lower than 10[kPa] at which the test subject feels generally the resistance.

A collecting efficiency obtained when this collecting part 2 was used and heptane was used as reference standard, was 50-70% sufficient for analyzing.

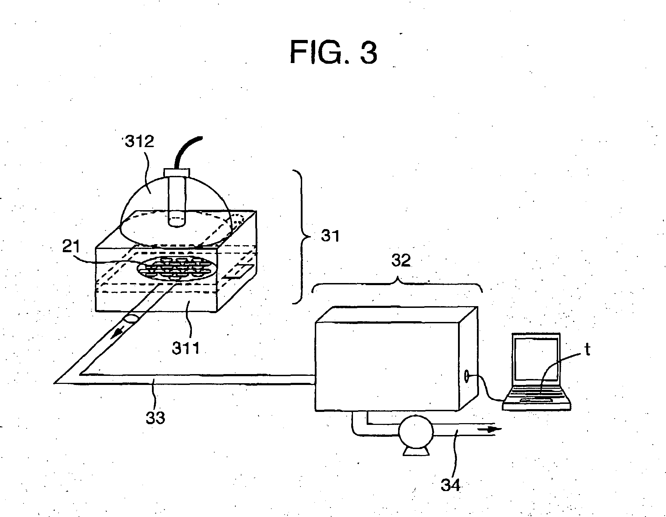

Fig. 3 is a schematic view showing a structure of an exhaled air analyzing system.

As shown in fig. 3, the exhaled air analyzing system 3 has a heating device 31 for containing the collecting filter 21 removed from the exhaled air collecting apparatus 1 (refer to fig. 1) and removing or vaporizing or reproducing the component of the exhaled air from the adsorbent 211 (refer to fig. 2) by heating, an analyzing device for analyzing the component of the exhaled air removed or vaporized or reproduced from the adsorbing filter 21 by being heated by the heating device 31, a pipe 33 connecting the heating device 31 and analyzing device to each other to transfer the component of the exhaled air removed or vaporized or reproduced by the heating device 31 to the analyzing device 32, and a discharge pipe 34 for discharging the component of the exhaled air analyzed by the analyzing device 32 to the outside of the system. The heating device has a heating chamber for containing the removed adsorbing filter 21, and a heating part 312 for heating the heating chamber 311. Further, the exhaled air analyzing system may has a terminal t to be electrically connected to the analyzing device 32 to analyze an analyzing result of the analyzing device 32. The heating part 312 includes, for example, a lamp light source, electric heater, an infra-red radiation heater or the like. The analyzing device 32 may have GC, ion-mobility, mass spectrometer, GC/MS (Gas Chromatography/Mass Spectrometer) or a combination thereof, but may have any device for analyzing the component.

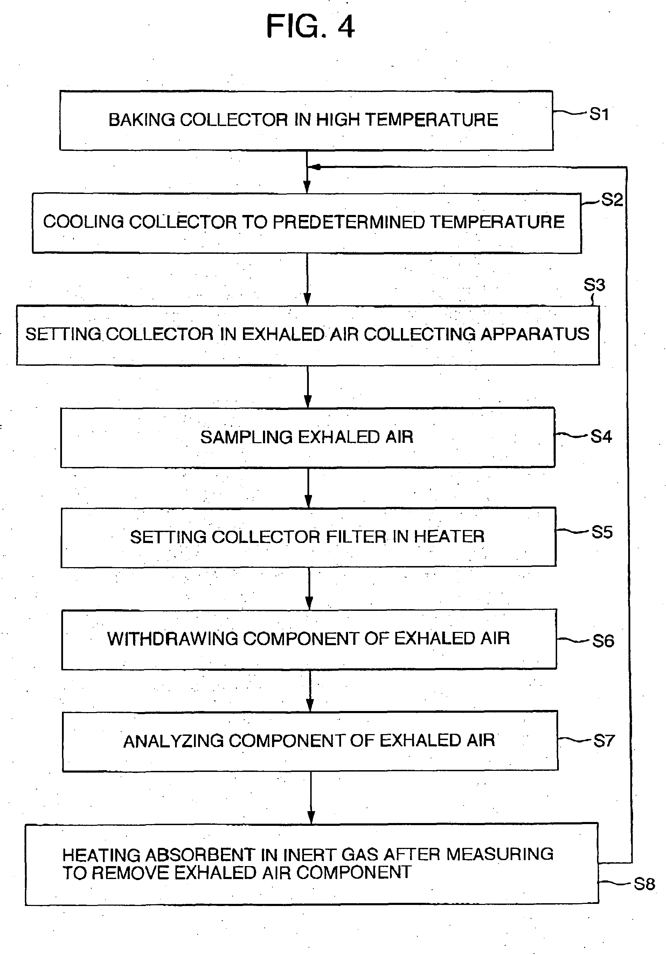

Fig. 4 is a flow chart showing a process for mesuring the component of the exhaled air.

The measuring process of the component of the exhaled air is explained along fig. 4 with making reference to figs. 1-3.

At first, the adsorbing part 2 is baked in high temperature (S1) to be pretreated for preventing unforeknown gas component from being adsorbed by the adsorbent 211. Next, it is cooled to a predetermined temperature in a clean gaseous environment of nitrogen, helium or the like (S2). The exhaled air receiving part 12 is attached onto the mouth of the test subject 11, and the exhaled air of the test subject 11 is sampled when the test subject 11 discharges the exhaled air into the exhaled air collecting apparatus 1 (S4). In this time, if the component of the exhaled air of high volatility needs to be measured, the component can be analyzed in high sensitivity by cooling the exhaled air collecting part 13 during the sampling to make the component effectively be adsorbed by a surface of the adsorbent 211. On the contrary, if the component of the exhaled air of low volatility needs to be measured, the component can be analyzed in high sensitivity by heating the exhaled air collecting part 13 to a temperature preventing the component from being removed from the exhaled air collecting part 13 during the sampling to make another component of high volatility not to be measured be restrained from being adsorbed by the adsorbent 211, so that an affection of noise component during analyzing is decreased. Incidentally, in this time, the exhaled air is collected while the amount of the exhaled air to be sampled is measured by a flow meter 15 mounted on the pipe 16a. The amount of the exhaled air to be sampled is preferably 1 1 / one sampling cycle. The amount of the exhaled air to be sampled is varied in accordance with the sensitivity of sensor and the component of the exhaled air to be measured.

After collecting the exhaled air, the adsorbing filter 21 is removed from the exhaled air collecting part 13, and mounted in the heating chamber 311 of the heating device 31 in the exhaled air analyzing system 3 (S5). The heating part 31 heats the heating chamber 311 to remove or vaporize or reproduce the component of the exhaled air from the adsorbing filter 21 so that the removed component of the exhaled air is collected (S6). For heating, the heat energy of, for example, the lamp light source is used. The collected component of the exhaled air is transferred to the analyzing device 32 through the pipe 33. The analyzing device 32 analyzes the transferred component of the exhaled air (S7). The analyzing device 32 can perform the analysis with the mass spectrograph, GC, GC/MS (Gas Chromatography/Mass Spectrometer) or the like.

The adsorbent 211 after finishing the measurement is heated in inert gas environment to remove completely the adsorbed component of the exhaled air (S8). By this treatment, the adsorbent 211 becomes reusable. If the sampling is not performed just after the heating, it is preferable that the adsorbent is contained in a hermetically sealed container to prevent the atmosphere from being adsorbed.

By arranging the adsorbent in such a manner that both $(AL - V)^2 / L^3 \geq 0.003$ [$mm^3$] and $V /Al \geq 0.3(30\%)$ are satisfied, the adsorbing filter by which the pressure loss is low, the load of the test subject is small, and a collecting efficiency of the component is high, is obtainable.

Table 1 shows a comparison between exhaled air collecting method as comparative examples and the exhaled air collecting method as the embodiment.

Here, the exhaled air collecting method are, for example, a method with using a container (bag) (for example, the method disclosed by the patent document 2) and a method with using adsorbent (for example, the method disclosed by the patent document 1), and the embodiment is the method with using the adsorbing filter.

On table 1, a relationship in the pressure loss of the adsorbing part 57 and the collecting efficiency of the component of the exhaled air among the exhaled air collecting methods of the method by collecting with the bag, the method by solid adsorption and the method by the adsorbing filter is shown.

Incidentally, the collecting efficiency of the component of the exhaled air is obtained from the experimental result using heptane as the reference standard.

table 1

| Exhaled air collecting method | Container (bag) collecting | Method with solid adsorption | Method with adsorbing filter |
|---|---|---|---|
| Pressure loss of adsorbing part | ≤ 1kPa | 40 kPa | ≤ 1kPa |
| Collecting efficiency of exhaled air component (experimental result with using heptane as reference specimen) | 10% | 90% | 50-70% |

As shown in table 1, in the method by container collecting, the pressure loss of the adsorbing part is not more than 1 [kPa] so that the load of the test subject is small, but the component collecting efficiency is about 10%, that is, low.

On the other hand, in the method by solid adsorption, the component collecting efficiency is about 90%, that is, high, but the pressure loss of the adsorbing part is 40 [kPa] so that the load of the test subject is great.

In the method with the adsorbing filter of the embodiment, the pressure loss of the adsorbing part is not more than 1 [kPa] so that the load of the test subject is small, and the component collecting efficiency is about 50-70%, that is, high, so that an improved sampling is obtainable.

An adsorbent temperature controller as shown in fig. 1 may heat the exhaled air to a temperature more than a temperature of the exhaled air received at the exhaled air receiving part 12 and/or heat the adsorbent to a temperature more than an atmospheric or environmental temperature and/or more than the temperature of the exhaled air received at the exhaled air receiving part 12, and/or may cool the exhaled air to a temperature less than a temperature of the exhaled air received at the exhaled air receiving part 12 and/or cool the adsorbent to a temperature less than the atmospheric or environmental temperature and/or less than the temperature of the exhaled air received at the exhaled air receiving part 12.

It should be further understood by those skilled in the art that although the foregoing description has been made on embodiments of the invention, the invention is not limited thereto and various changes and modifications may be made without departing from the spirit of the invention and the scope of the appended claims.

**Claims**

1. A filter for collecting a component of an exhaled air, comprising,
an adsorbent (211) for adsorbing the component of the exhaled air, and
a collector body (2) including a passage hole (213) in which the adsorbent is contained and through which the exhaled air is capable of passing.

2. A filter according to claim 1, wherein when a total amount of volume of the adsorbent is V, a total amount of cross sectional area of the passage hole is A, and a length of the passage hole is L, $(AL - V)^2/L^3 \geq 0.003mm^3$, and $V/AL \geq 0.3$.

3. A filter according to claim 1 or 2, wherein the collector body has a pair of meshes (22) covering respective opening ends of the passage hole to prevent the adsorbent from moving out of the passage hole and to allow the exhaled air to pass through the meshes.

4. A filter according to any one of claims 1-3,
wherein the collector body includes a plurality of the passage holes arranged in parallel in a flow direction of the exhaled air so that the passage holes are capable of passing respective parts of the exhaled air in parallel.

5. A filter according to claim 4, wherein the collector body has a partition wall between the passage holes to prevent the parts of the exhaled air in the respective passage holes from fluidly communicating with each other in a direction perpendicular to the flow direction of the exhaled air in the collector body.

6. A filter according to claim 4 or 5, wherein the collector body has a pair of meshes (22) covering respective opening ends of each of the passage holes to prevent the adsorbent from moving out of the passage hole and to allow the exhaled air to pass through the meshes, and each of the meshes extends monolithically to cover the opening ends of the passage holes.

7. A filter according to any one of claims 1-6,
wherein the collector body is metallic.

8. A filter according to any one of claims 1-7,
wherein the adsorbent includes a carbon type adsorbent.

9. An apparatus for collecting an exhaled air of a test subject, comprising,
an inlet (12) for receiving the exhaled air from the test subject, and a filter for collecting a component of the exhaled air,
wherein the filter includes an adsorbent (211) for adsorbing the component of the exhaled air, and a collector body (2) including a passage hole in which the adsorbent is contained and through which the exhaled air is capable of passing.

10. An apparatus according to claim 9, wherein when a total amount of volume of the adsorbent is V, a total amount of cross sectional area of the passage hole is A, and a length of the passage hole is L, $(AL - V)^2/L^3 \geq 0.003mm^3$, and $V/AL \geq 0.3$.

11. An apparatus according to claim 9 or 10,
wherein the collector body has a pair of meshes covering respective opening ends of the passage hole to prevent the adsorbent from moving out of the passage hole and to allow the exhaled air to pass through the meshes.

12. An apparatus according to any one of claims 9-11, further comprising a flow meter for measuring a flow rate of the exhaled air passing through the passage hole to measure a total amount of the exhaled air passing through the passage hole.

13. An apparatus according to any one of claims 9-12, further comprising a suction pump fluidly connected to one of opening ends of the passage hole to accelerate a flow of the exhaled air from the other one of the opening ends toward the one of the opening ends.

14. An apparatus according to any one of claims 9-13, further comprising a heater for heating the adsorbent to remove the component from the adsorbent, and an analyzer for analyzing the component removed from the adsorbent.

15. An apparatus according to claim 14, wherein the heater is capable of generating a radiant heat for irradiating the filter.

16. An apparatus according to claim 14 or 15,
wherein the analyzer includes at least one of a mass spectrometer and a gas chromatograph.

17. A method for analyzing an exhaled air, in an apparatus for collecting an exhaled air, including an inlet for receiving the exhaled air from a test subject, and a filter for collecting a component of the exhaled air, wherein the filter includes an adsorbent for adsorbing the component of the exhaled air, and a collector body including a passage hole in which the adsorbent is contained and through which the exhaled air is capable of passing, comprising the steps of:

receiving the exhaled air at the inlet so that the exhaled air is allowed to pass through the passage hole,
heating the adsorbent to remove the component from the adsorbent, and
analyzing the removed component.

18. A method according to claim 17, further comprising the step of
heating the adsorbent before the step of receiving the exhaled air at the inlet.

19. A method according to claim 17 or 18, wherein a temperature of the adsorbent is kept at a temperature more than a temperature of the exhaled air received at the inlet, in the step of receiving the exhaled air.

20. A method according to any one of claims 17-19, wherein a temperature of the adsorbent is kept at a temperature less than a temperature of the exhaled air received at the inlet, in the step of receiving the exhaled air.

## FIG. 1

## FIG. 2A

EXHALED AIR

## FIG. 2B

# FIG. 3

# FIG. 4

| BAKING COLLECTOR IN HIGH TEMPERATURE | S1 |

| COOLING COLLECTOR TO PREDETERMINED TEMPERATURE | S2 |

| SETTING COLLECTOR IN EXHALED AIR COLLECTING APPARATUS | S3 |

| SAMPLING EXHALED AIR | S4 |

| SETTING COLLECTOR FILTER IN HEATER | S5 |

| WITHDRAWING COMPONENT OF EXHALED AIR | S6 |

| ANALYZING COMPONENT OF EXHALED AIR | S7 |

| HEATING ABSORBENT IN INERT GAS AFTER MEASURING TO REMOVE EXHALED AIR COMPONENT | S8 |

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10206293 A
- JP 2003171317 A
- JP 8051098 A